# EUROPÄISCHE PATENTSCHRIFT

(11) **EP 1 023 036 B1**
(45) Veröffentlichungstag und Bekanntmachung des Hinweises auf die Patenterteilung: **09.01.2002**
(21) Anmeldenummer: 98958205.1
(22) Anmeldetag: 15.10.1998
(51) Int. Cl.: A61K 7/22, A61K 7/16

(54) **DESINFEKTIONSMITTEL**
DISINFECTING AGENT
DESINFECTANT

(30) Priorität: 18.10.1997 DE 19746205; 23.02.1998 DE 19807433
(43) Veröffentlichungstag der Anmeldung: 02.08.2000
(73) Patentinhaber: DDG Dental Devices GmbH, 20537 Hamburg (DE)
(72) Erfinder: INGEMANN, Knut, D-21227 Bendestorf (DE)
(74) Vertreter: Schupfner, Georg U.
(86) Internationale Anmeldenummer: DE9803084
(87) Internationale Veröffentlichungsnummer: WO9920228

(56) Entgegenhaltungen:
- EP-A- 0 244 363
- EP-A- 0 363 748
- WO-A-85/01876
- WO-A-87/05501
- WO-A-95/12395
- WO-A-97/13495

## Beschreibung

Die Erfindung betrifft eine Desinfektionsmittel-Zusammensetzung enthaltend Chlorphenyl- und/oder Chlorbenzyl-Biguanid-Verbindungen und Benzoesäure bzw. deren Salze in wäßriger alkoholischer Lösung und insbesondere deren Verwendung für den Dentalbereich.

Die Verwendung von Biguanid-Verbindungen wie Chlorhexidin als Desinfektionsmittel ist an sich bekannt. Chlorhexidin (1,1'-Hexamethylenbis[5-(4-chlorphenyl)-Biguanid] oder auch N,N"-bis(4-chlorophenyl)-3,12-diimino)-2,4,11,13-Tetraazatetradecandiimidamid) ist ein auch im Mundraum antiseptisch wirkendes Desinfektionsmittel mit folgender Struktur:

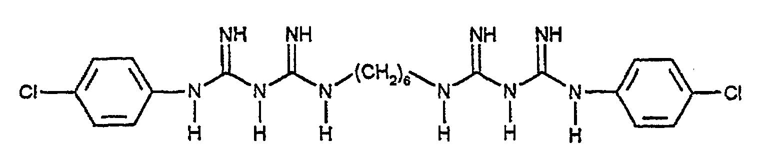

Chlorhexidin besitzt stark basische Gruppen mit Guanidin-Struktur. Es ist nur wenig toxisch und wird auch von den Schleimhäuten gut vertragen. Vom Chlorhexidin sind weiterhin verschiedene Derivate bekannt. Genannt seien u.a. das Diacetat, das Digluconat oder das Chlorhydrat. Von dem Diacetat ist bekannt, daß es in Wasser löslich ist. Es ist weiterhin aus der EP 0 185 971 bekannt, daß die antimikrobielle Wirksamkeit von Biguanid-Verbindungen gegen grampositive Keime durch die Anwesenheit von Alkylglykosid-Tensiden gesteigert wird.

Aus der WO 87/05501 ist Mundwasser enthaltend ein Salz der Benzoesäure und Chlorhexidinegluconat in wäßriger ethanolischer Lösung bekannt. Die dort offenbarte Zusammensetzung ist jedoch nicht lagerstabil und weist nicht die Eigenschaften auf, die ein Desinfektionsmittel aufweisen muß, wenn es zur Desinfektion von Zahnbürsten oder anderen Gegenständen außerhalb des Mundraumes eingesetzt werden soll.

Aufgabe der vorliegenden Erfindung ist es ein Desinfektionsmittel für den Dentalbereich zur Anwendung außerhalb des Mundraumes bereitzustellen, daß gleichzeitig eine ausgezeichnete fungizide, bakerizide und viruzide Wirkung hat, kurzfristig keimtötend wirkt, ein breites Wirkungsspektrum zeigt, eine geringe Toxizität aufweist, keinerlei Einwirkungen auf das Material der zu desinfizierenden Gegenstände zeigt und lagerstabil ist.

Die Aufgabe wurde überraschend erfindungsgemäß gelöst durch eine Desinfektionsmittel-Zusammensetzung mit einem pH von 5 bis 9, insbesondere 5 bis 8, enthaltend:
(a) 0,001 bis 4 Gew.% , vorzugsweise 0,01 bis 2 Gew.%, besonders bevorzugt 0,6 bis 2 Gew.%, bezogen auf die freie Base, einer oder mehrerer Chlorphenyl- und/oder Chlorbenzyl-Biguanid-Verbindungen,
(b) 0,005 bis 7 Gew.% , vorzugsweise 0,01 bis 3 Gew.%, besonders bevorzugt 0,5 bis 3 Gew.%, bezogen auf die freie Säure, Benzoesäure und/oder ein Salz der Benzoesäure, sowie
(c) mehr als 25 Gew.%,, vorzugsweise mehr als 35 Gew.%, insbesondere 35 bis 95 Gew.% bzw. 35 bis 75 Gew.%, eines C2- bis C4- Alkohols und
(d) Wasser, vorzugsweise 0,5 bis 65 Gew.%, besonders bevorzugt 25 - 65 Gew.%, Wasser.

Weiterhin können in der Desinfektionsmittel - Zusammensetzung unabhängig voneinander
(e) 0,01 - 8 Gew.%, vorzugsweise 0,05 - 6 Gew.%, insbesondere 1,0 - 5 Gew.%, nicht-ionische Tenside,
(f) 0,01 - 5 Gew.%, vorzugsweise 1 - 5 Gew.%, Ätherische Öle / Fruchtaromen, insbesondere 0,02 bis 4 Gew.% bzw. 2 - 4 Gew.% Thymol und/oder Menthol, und
(g) 0 - 3 Gew.% Süßstoffe
enthalten sein.

Die Chlorphenyl-Biguanid-Verbindung (a) ist vorzugsweise Chlorhexidin oder ein Chlorhexidin-Salz, besonders bevorzugt Chlorhexidindiacetat und/oder Chlorhexidindigluconat. Nach einer bevorzugten Ausführungsform der Erfindung kann die Desinfektionsmittelzusammensetzung z.B. wie folgt zusammengesetzt sein:
(a) 0,01 bis 2 Gew.%, vorzugsweise 1 bis 2 Gew.%, Chlorhexidindiacetat und/oder Chlorhexidindigluconat,
(b) 0,01 bis 3 Gew.%, vorzugsweise 0,5 bis 3 Gew.%, Benzoesäure oder eines Salzes der Benzoesäure,
(c) 25 bis 75 Gew.%, vorzugsweise 30 bis 60 Gew.%, des Alkohols und
(d) 25 bis 70 Gew.%, vorzugsweise 40 bis 70 Gew.%, Wasser.

Für kleine Einsatzmengen Chlorphenyl- und/oder Chlorbenzyl-Biguanid-Verbindungen hat sich folgende Desinfektionsmittel-Zusammensetzung als besonders wirksam erwiesen
(a) 0,001 bis kleiner 0,1 Gew.%, bezogen auf die freie Base, einer oder mehrerer Chlorphenyl- und/oder Chlorbenzyl-Biguanid-Verbindungen,
(b) 0,005 bis kleiner 0,1 Gew.%, bezogen auf die freie Säure, Benzoesäure oder eines Salzes der Benzoesäure,
(c) 35 - 65 Gew.% des Alkohols und
(d) 35 - 65 Gew.% Wasser

Als geeignete C2- bis C4- Alkohole haben sich Ethanol, n-Propanol und/oder Isopropanol erwiesen.

Ätherische Öle / Fruchtaromen gemäß der vorliegenden Erfindung sind beispielsweise Menthol, Thymol, Eucalyptol, Anethol Carvon, Anethol, Cineol, Eugenol, Zimtaldehyd, Caryophyllen, Geraniol, Citronellol, Linaleol, Salven, Tymol, Terpinen, Methylchavicol, Methylsalicylat, Mentylacetat, Vanillin, Jonone, Linalylacetat, Rhodinol, Piperiton oder deren Mischungen, insbesondere Menthol oder Thymol. Vorzugsweise enthält die Zusammensetzung Menthol.

Geeignete Süßstoffe sind beispielsweise: Saccharin-Natrium (Natriumsalz der 1,2-Benisothiazol-3(2H)-on-1,1-dioxides, Natriumsalz des Benzolsulfimides), Cyclamate, Acesulfam-Kalium, Aspartame ®, Glycerin, Sorbit, Mannit und Xylit, vorzugsweise solche ohne kariogene Wirkung.

Besonders bevorzugt ist das in der Desinfektionslösung enthaltende Tensid ein Alkyl-/Alkenylglykosid, ein oxalkylierter Alkohol, eine oxalkylierte Carbonsäure, ein Sorbitanester, ein polyoxyethyliertes Derivat eines Sorbitanesters (insbesondere die Tween®- und Span® Handelsprodukte), ein Polyglycerinester, ein Succroat (Fettsäureester der Saccharose), ein Ester einer Fettsäure mit einem Polyalkohol und/oder ein Polyalkylenglykol. Das Tensid kann insbesondere ein Alkylglykosid sein.

Die bevorzugten Alkyl- / Alkenylglykoside sind aus Fettalkoholen und aus (ringförmigen, beliebigen) Zuckermolekülen abgeleitet und weisen insbesondere Glukose- und/ oder Maltose-Einheiten, ggf, glykosidisch gebunden, und ein mittleres Molekulargewicht von 250 bis 1000 g/mol, vorzugsweise von 300 bis 500 g/mol, auf und zumindest einem Kohlenwasserstoffrest mit 6 bis 20, vorzugsweise 8 bis 16 Kohlenstoffatomen.

Die Alkyl- / Alkenylglykoside können oligomer sein, wobei der Glykosid-Anteil im Mittel aus ein bis drei, vorzugsweise ein bis zwei Glykosid-Einheiten besteht. Die Alkenylglykoside können ein oder zwei Doppelbindungen aufweisen.

Ein typisches Monoglykosid im vorliegenden Zusammenhang ist z.B.:

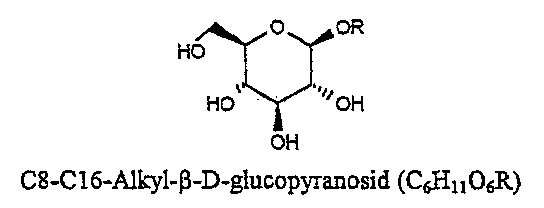

Erfindungsgemäß eingesetzte Alkylglykoside sind z.B. die Handelsprodukte Plantacare® der Henkel KGaA (z.B. Typ 818 UP enthaltend 50% Aktivsubstanz und 3-5 % Polyglucose in wäßriger Dispersion, neutralisiert mit einer organischen Säure wie Essigsäure).

Nach einer weiteren bevorzugten Ausführungsform der Erfindung enthält die Desinfektionsmittel - Zusammensetzung:
(e) 1 bis 3 Gew.% Alkyl- / Alkenylglykoside als Tensid und
(f) 2 bis 4 Gew.% Thymol und/oder Menthol, vorzugsweise 2,5 bis 4,0 Gew.% Menthol, als Ätherische Öle / Fruchtaromen.

Die Desinfektionsmittel - Zusammensetzung wird vorzugsweise auf einen pH von 5 bis 8, vorzugsweise etwa 7, eingestellt. Weitere an sich bekannte Additive wie Verdicker, Konservierungsstoffe, Färbemittel, Antioxidantien, Geruchsstoffe, Geschmacksverbesserer, Flourid-Ionen Spender, Schaumbildner können ebenso in der Zusammensetzung enthalten sein.

Es kann z.B. vorteilhaft sein die Desinfektionsmittel-Zusammensetzung in Form eines Gels, Schaums oder Sprays einzusetzen. Eine solche Zusammensetzung enthält dann neben der oben beschriebenen Desinfektionsmittel - Zusammensetzung zusätzlich 10 bis 90 Gew.%, vorzugsweise 20 bis 60 Gew.% eines Treibmittel, wie Dimethylether, Kohlendioxid, Propan, Butan oder einen Halogenkohlenwasserstoff, wie einen Fluorchlorkohlenwasserstoff oder Fluorkohlenwasserstoff, oder 0,1 bis 10 Gew.%, vorzugsweise 0,2 bis 2 Gew.%, eines Verdickungsmittels (Geliermittels) wie Zellulose oder chemisch modifizierte Zellulose-Derivate wie Hydroxypropylcellulose, Hydroxyethylcellulose oder wasserlösliche Salze von Zelluloseethern, biologisch hergestellte Vielfachzucker (z.B. Xanthene), Polyvinylalkohole, Copolymere der Maleinsäure mit Vinylmonomeren, Polyacrylsäure oder deren Salze, Polyarylamide, Carbopol® oder kationische Polymere, wie z.B. Flotationshilfsmittel.

Zahnbürsten werden im Mundraum zur Reinigung von Zähnen eingesetzt. Die Zahnbürste wird in der Regel in ihrem mechanischen Reinigungseffekt von Zahnpflegemitteln unterstützt, um Ansammlungen von Speiseresten und Bakterienbelägen in der Mundhöhle so weit wie möglich zu verhindern. Zahnpflegemittel wie Zahncremes können Desinfektionsmittel enthalten. Auch der Gebrauch von Desinfektionsmitteln in Mundwässern ist bekannt. Die ständige Einwirkung von desinfizierenden Maßnahmen bei Einsatz von Bakterioziden in Mundpflegemitteln kann die physiologische Mundflora schwächen. Die erfindungsgemäße Desinfektionsmittel - Zusammensetzung ist hinsichtlich ihrer Zusammensetzung für den Einsatz außerhalb des Mundraumes konzipiert. Dort kann der Anwendung entsprechend keine Schwächung der Mundflora auftreten.

Die erfindungsgemäße Desinfektionsmittel-Zusammensetzung wird vorzugsweise zur Desinfektion von Gegenständen eingesetzt, die - etwa zur Zahnpflege - mehr als einmal in den Mundraum geführt werden. Gegenstände dieser Art sind etwa Zahnbürsten, Zahnstocher, Zahnspangen, Zahnschienen, Zahnschutz, Aufsätze für Mundduschen (Düsen) oder auch Zahnprothesen. Diese Gegenstände sind beim Zahnreinigungsvorgang den Bakterien, Viren oder Fungen des Mundraumes ausgesetzt und werden nach der Anwendung oft nur unzureichend mit Wasser gereinigt. Diese Reinigung ist insbesondere aus mikrobiologischer Sicht unzureichend. Zahnbürsten etwa können ein idealer Nährboden für Bakterien und Pilze sein, wenn sie nach der Reinigung mit Wasser etwa bei Raumtemperatur mehrere Stunden stehen und sodann erneut eingesetzt werden. Viren können über Tage hinweg infektiös bleiben. Besonders vorteilhaft wird die Desinfektionsmittel-Zusammensetzung in einer Vorrichtung gemäß der WO 98/01054 eingesetzt.

Die Wirksamkeit der Desinfektionsmittel-Zusammensetzung für die oben erwähnten Anwendungen wurden anhand verschiedener mikrobiologischer Test nachgewiesen. Die Konzentrationsangaben beziehen sich jeweils auf den aktiven Wirkstoff.

### Beispiele

### Desinfektionsmittel-Zusammensetzung:

| | Gew.% |
|---|---|
| Chlorhexidindiacetat | 1,50 |
| Benzoesäure | 2,00 |
| Thymol DAB 10 | 0,10 |
| Menthol DAB 10 | 3,00 |
| Natrium-Saccarinat | 0,30 |
| Laurylpolyglycosid neutralisiert mit Essigsäure | 2,00 |
| Ethanol | 47,21 |
| Aqua purificata | 43,89 |

### Mikrobiologischer Test:

Die Desinfektionsmittel-Zusammensetzung wurde hinsichtlich ihrer bakteriziden und fungiziden Wirksamkeit ausgetestet, um zu ermitteln, ob die Desinfektionsmittel-Zusammensetzung als Lösung für die praktische Anwendung geeignet ist.

Dazu wurden Konservierungstests u.a. mit Candida albicans durchgeführt. Der Konservierungstest zeigte nach 24 Stunden kein Wachstum von Candida albicans. Als Testkeime wurden nach ATCC verwendet: Pseudomonas aeruginosa und Candida albicans. Hierzu wurden die Zahnbürsten mit einer Keimkonzentration von 10⁵ benetzt und anschließend mit zwei Sprühstößen der Desinfektionsmittel-Zusammensetzung besprüht. Die Zahnbürsten wurden nach 15, 30 und 60 Minuten Einwirkzeit in Peptonwasser ausgewaschen und filtriert. Sodann wurden die Filter 24 Stunden auf TSG Aga bebrütet. Es zeigten sich weder nach 15 Minuten noch nach 30 und 60 Minuten bei Pseudomonas aeruginosa ein Wachstum der Keime (Tabelle 2).

Bei Candida albicans zeigte sich nach 15 und 30 Minuten noch Wachstum, nach 60 Minuten Einwirkzeit der Desinfektionsmittel-Zusammensetzung lediglich das Wachstum von 2 Kolonien in den Proben, die mit der Desinfektionsmittel-Zusammensetzung besprüht waren.

Somit ist eine deutliche Reduktion um mindestens vier Logstufen gegeben. Die weitere Austestung gegen diverse Keime wurde ebenso mit Desinfektionsmittel-Zusammensetzung vorgenommen. Dazu wurden ATCC-Stämme der Spezies Staph. aureus, Candida albicans, hämolysierende Streptokokken der Gruppe B, Klebsiella pneumoniae ausgetestet. Hierzu wurden ebenfalls die Zahnbürsten mit einer Keimkonzentration von 10⁵ benetzt und mit zwei Sprühstößen des Mundwassers 11 versehen. Die Einwirkzeit des Mundwassers betrug hier standardisiert 15, 30, 60 Minuten, 2 Stunden und 4 Stunden.

Auch hier wurden die Zahnbürsten wieder in Peptonwasser ausgespült, die Lösung über einen Sterilfilter gegeben und anschließend auf TSD Aga 24 Stunden bebrütet. Der Keimnachweis ist Tabelle 2 zu entnehmen. Es zeigt sich, daß die Pilzkonzentrationen nach 60 Minuten nur noch sehr vereinzelt noch nachweisbar war. Die Keimkonzentrationen waren jedoch bei den anderen Keimen spätestens nach 60 Minuten nicht mehr anzüchtbar. Die beta-hämolysierenden Streptokokken waren bereits nach 5 Minuten nicht mehr anzüchtbar. Klebsiella pneumoniae war ebenfalls nach 60 Minuten nicht mehr anreicherungsfähig.

Die durchgeführten mikrobiologischen Versuche zeigen, daß die Desinfektionslösungen dazu geeignet sind, Bakterien, Pilze und Viren nach einer entsprechenden Einwirkzeit zu beseitigen. Die Austestung mit den Desinfektionsmittel-Zusammensetzungen bei verschiedenen Einwirkzeiten erbrachte, daß bei einer Stehzeit von 60 Minuten die Keime in dem Desinfektionsbehälter für Zahnbürsten abgetötet sind.

Candida ist erfahrungsgemäß resistenter und läßt sich innerhalb von 60 Minuten von einer Logstufe 10⁵ auf < 10¹ reduzieren. Die in der Tabelle dargestellten Ergebnisse wurden ermittelt, ohne die Zahnbürste vorher einer Zahncreme mit entsprechender bakteriostatischer Wirkung auszusetzen. Die Desinfektionsmittel-Zusammensetzung ist geeignet, eine bakteriell kontaminierte Zahnbürste ausreichend zu desinfizieren. In den meisten Fällen reicht bereits eine Stehzeit von 5 - 30 Minuten aus, um die Keime um mindestens 3 Logstufen zu reduzieren.

Vergleichsbeispiele ohne Einsatz von Benzoesäure zeigen, daß die fungizide Wirkung der Desinfektionsmittel-Zusammensetzung stark vermindert wird und die Zusammensetzung nicht mehr zur wirkungsvollen Desinfektion von Zahnbürsten eingesetzt werden kann.

Als wirksam zur Zahnbürstendesinfektion haben sich auch folgende Desinfektionsmittel-Zusammensetzungen (A) und (B) erwiesen, enthaltend im wesentlichen:

| | (A) Gew.% | (B) Gew.% |
|---|---|---|
| Chlorhexidin als Base gereicht | 0,02 | 0,03 |
| Benzoesäure | 0,03 | 0,03 |
| Ethanol | 52 | 48 |
| Rest jeweils Aqua purificata, Tenside und weitere Hilfsstoffe | | |

## Patentansprüche

1. Desinfektionsmittel-Zusammensetzung geignet für Gegenstände, die mehr als einmal in den Mundraum geführt werden, mit einem pH von 5 bis 9 enthaltend:
(a) 0,01 bis 2 Gew.% einer oder mehrerer Chlorphenyl- und/oder Chlorbenzyl-Biguanid-Verbindungen, bezogen auf die freie Base,
(b) 0,005 bis 7 Gew.% Benzoesäure und/oder ein Salz der Benzoesäure, bezogen auf die freie Säure, sowie
(c) wenigstens 25 Gew.%, vorzugsweise wenigstens 35 Gew.%, eines C2- bis C4- Alkohols,
(d) Wasser und
(f) 0,01 bis 5 Gew.% ätherische Öle / Fruchtaromen.

2. Desinfektionsmittel-Zusammensetzung gemäß Anspruch 1, **dadurch gekennzeichnet, daß** die Chlorphenyl-Biguanid-Verbindung (a) Chlorhexidin oder ein Chlorhexidin-Salz, vorzugsweise Chlorhexidindiacetat und/oder Chlorhexidindigluconat, ist.

3. Desinfektionsmittel-Zusammensetzung gemäß einem der vorhergehenden Ansprüche enthaltend unabhängig voneinander:
(c) 35 bis 95 Gew.% eines C2- bis C4- Alkohols und
(d) 0,5 bis 65 Gew.% Wasser.

4. Desinfektionsmittel-Zusammensetzung gemäß einem der vorhergehenden Ansprüche, **dadurch gekennzeichnet, daß** die Zusammensetzung enthält:
(a) 0,6 bis 2 Gew.%, vorzugsweise 1 bis 2 Gew.%, Chlorhexidindiacetat und/oder Chlorhexidindigluconat,
(b) 0,01 bis 3 Gew.%, vorzugsweise 0,5 bis 3 Gew.%, Benzoesäure oder eines Salzes der Benzoesäure,
(c) 25 bis 75 Gew.%, vorzugsweise 30 bis 60 Gew.%, des Alkohols und
(d) 25 bis 70 Gew.%, vorzugsweise 40 bis 70 Gew.%, Wasser.

5. Desinfektionsmittel-Zusammensetzung gemäß einem der vorhergehenden Ansprüche, **dadurch gekennzeichnet, daß** die Zusammensetzung weiterhin enthält:
(g) 0 bis 3 Gew.% Süßstoffe.

6. Desinfektionsmittel-Zusammensetzung gemäß einem der vorhergehenden Ansprüche, **dadurch gekennzeichnet, daß** die Zusammensetzung weiterhin enthält:
(f) 0,02 bis 4 Gew.% Thymol und/oder Menthol als ätherische Öle / Fruchtaromen.

7. Desinfektionsmittel-Zusammensetzung gemäß einem der vorhergehenden Ansprüche, **dadurch gekennzeichnet, daß** die Zusammensetzung weiterhin enthält
(e) 0,01 bis 8 Gew.%, vorzugsweise 1 bis 5 Gew. %, eines oder mehrerer nicht-ionischer Tenside.

8. Desinfektionsmittel-Zusammensetzung gemäß Anspruch 7, dadarch **gekennzeichnet**, daß das Tensid (e) ein Alkyl- / Alkenylglykosid, ein oxalkylierter Alkohol, eine oxalkylierte Carbonsäure, ein Sorbitanester, ein polyoxyethyliertes Derivat eines Sorbitanesters, ein Polyglycerinester, ein Ester einer Fettsäure mit einem Polyalkohol und/oder ein Polyalkylenglykol ist.

9. Desinfektionsmittel-Zusammensetzung gemäß einem der Ansprüche 1 bis 7, **dadurch gekennzeichnet, daß** die Zusammensetzung enthält:
(e) 1 bis 3 Gew.% Alkyl- / Alkenylglykoside als Tensid
(f) 2 bis 4 Gew.% Thymol und/oder Menthol als ätherische Öle / Fruchtaromen.

10. Desinfektionsmittel-Zusammensetzung gemäß einem der Ansprüche 7 oder 9, **dadurch gekennzeichnet, daß** das Tensid (e) ein Alkyl- / Alkenylglykosid mit einem mittleren Molekulargewicht von 250 bis 1000 g/mol, vorzugsweise von 300 bis 500 g/mol, ist und zumindest einen Kohlenwasserstoffrest mit 6 bis 20, vorzugsweise 8 bis 16 Kohlenstoffatomen aufweist.

11. Desinfektionsmittel-Zusammensetzung gemäß einem der vorhergehenden Ansprüche, **dadurch gekennzeichnet, daß** die wäßrige alkoholische Zusammensetzung einen pH von 5 bis 8, vorzugsweise von etwa 7, aufweist.

12. Desinfektionsmittel-Zusammensetzung gemäß einem der vorhergehenden Ansprüche, **dadurch gekennzeichnet, daß** die Zusammensetzung als Alkohol (c) Ethanol, n-Propanol und/oder Isopropanol enthält.

13. Desinfektionsmittel - Zusammensetzung nach einem der vorhergehenden Ansprüche, **dadurch gekennzeichnet, daß** sie 2,5 bis 4,0 Gew.% Menthol enthält.

14. Zusammensetzung, **dadurch gekennzeichnet, daß** diese die Desinfektionsmittel-Zusammensetzung nach einem der vorherigen Ansprüche zusammen mit 10 bis 90 Gew.% eines Treibmittels oder 0,1 bis 10 Gew.% eines Verdickungsmittels enthält.

15. Gegenstände, die geeignet sind, zum Zwecke der Zahnpflege, des Zahnersatzes oder der Mundhygiene, insbesondere Zahnbürsten, mehr als einmal in den Mundraum geführt zu werden, benetzt mit einer Desinfektionsmittel-Zusammensetzung gemäß einem der Ansprüche 1 bis 13 oder einer Zusammensetzung gemäß Anspruch 14.

16. Verwendung der Desinfektionsmittel-Zusammensetzung gemäß einem der Ansprüche 1 bis 13 oder der Zusammensetzung gemäß Anspruch 14 zur Desinfektion von Gegenständen, die zum Zwecke der Zahnpflege, des Zahnersatzes oder der Mundhygiene, insbesondere von Zahnbürsten, mehr als einmal in den Mundraum geführt werden.

## Claims

1. A disinfectant composition suitable for objects to be introduced into the oral cavity more than once, having a pH of 5 to 9 containing:
a) 0,01 to 2 wt% of one or more chlorophenyl biguanide and/or chlorobenzyl biguanide compounds based on the free base,
b) 0,005 to 7 wt% benzoic acid and/or a salt of benzoic acid, based on the free acid, and
c) at least 25 wt%, preferably at least 35 wt% of a C2- to C4- alcohol, and
d) water, and
e) 0,01 to 5 wt% essential oils / fruit flavorings.

2. Disinfectant composition according to claim 1, **characterized in that** the chlorophenyl biguanide compound (a) is chlorhexidine or a chlorhexidine salt, preferably chlorhexidine diacetate and/or chlorhexidine digluconate.

3. Disinfectant composition according to one of the preceding claims containing, independently from one another:
c) 35 to 95 wt% of a C2- to C4- alcohol and
d) 0,5 to 65 wt% water.

4. Disinfectant composition according to one of the preceding claims, **characterized in that** the composition contains:
a) 0,6 to 2 wt%, preferably 1 to 2 wt% chlorhexidine diacetate and/or chlorhexidine digluconate,
b) 0,01 to 3 wt%, preferably 0,5 to 3 wt% benzoic acid or a salt of benzoic acid,
c) 25 to 75 wt%, preferably 30 to 60 wt%, of said alcohol and
d) 25 to 70 wt%, preferably 40 to 70 wt% water.

5. Disinfectant composition according to one of the preceding claims, **characterized in that** the composition further contains:
g) 0 to 3 wt% sweeteners.

6. Disinfectant composition according to one of the preceding claims, **characterized in that** the composition further contains:
f) 0,02 to 4 wt% thymol and/or menthol as essential oils / fruit flavorings.

7. Disinfectant composition according to one of the preceding claims, **characterized in that** the composition further contains:
e) 0,01 to 8 wt%, preferably 1 to 5 wt% of one or more nonionic surfactants.

8. Disinfectant composition according to claim 7, **characterized in that** the surfactant(e) is an alkyl glycoside / alkenyl glycoside, an alkoxylated alcohol, an alkoxylated carboxylic acid, a sorbitan ester, a polyethoxylated derivative of a sorbitan ester, a polyglycerol ester, an ester of a fatty acid with a polyalcohol and/or a polyalkylene glycol.

9. Disinfectant composition according to one of claims 1 through 7, **characterized in that** the composition contains:
e) 1 to 3 wt% alkyl glycosides or alkenyl glycosides as surfactant,
f) 2 to 4 wt% thymol and/or menthol as essential oils or fruit flavorings.

10. Disinfectant composition according to one of claims 7 or 9, **characterized in that** the surfactant(e) is an alkyl glycoside / alkenyl glycoside with an average molecular weight of 250 to 1000 g/mol, preferably 300 to 500 g/mol, and comprises at least one hydrocarbon residue with 6 to 20 carbon atoms, preferably 8 to 16 carbon atoms.

11. Disinfectant composition according to one of the preceding claims, **characterized in that** the aqueous alcoholic composition has a pH of 5 to 8, preferably of about 7.

12. Disinfectant composition according to one of the preceding claims, **characterized in that** the composition as alcohol (c) contains ethanol, n-propanol and/or isopropanol.

13. Disinfectant composition according to one of the preceding claims, **characterized in that** it contains 2,5 to 4,0 wt% menthol.

14. Composition **characterized in that** it contains the disinfectant composition according to one of the preceding claims together with 10 to 90 wt% of a blowing agent or 0,1 to 10 wt% of a thickener.

15. Objects which are suitable to be introduced into oral cavity more than once for the purpose of dental care, dental prostheses or oral hygiene, in particular toothbrushes, wetted with a disinfectant composition according to one of claims 1 to 13 or a composition according to claim 14.

16. Use of the disinfectant composition according to one of claims 1 through 13 or the composition according to claim 14 for disinfection of objects which are introduced more than once in the oral cavity for the purpose of dental care, dental prostheses (care) or oral hygiene, in particular toothbrushes.

## Revendications

1. Composition désinfectante ayant un pH de 5 à 9, convenant pour des objets qui sont introduits plus d'une fois dans la cavité buccale, contenant :
(a) 0,01 à 2 % en poids d'un ou plusieurs composés de chlorophényl- et/ou de chlorobenzyl-biguanide, par rapport à la base libre,
(b) 0,005 à 7 % en poids d'acide benzoïque et/ou d'un sel d'acide benzoïque, par rapport à l'acide libre, ainsi que
(c) au moins 25 % en poids, de préférence au moins 35 % en poids, d'un alcool en C₂ à C₄,
(d) de l'eau et
(f) 0,01 à 5 % en poids d'huiles essentielles/arômes de fruits.

2. Composition désinfectante suivant la revendication 1, **caractérisée en ce que** le composé de chlorophényl-biguanide (a) est la chlorhexidine ou un sel de chlorhexidine, de préférence le diacétate de chlorhexidine et/ou le digluconate de chlorhexidine.

3. Composition désinfectante suivant l'une des revendications précédentes, contenant, indépendamment l'un de l'autre :
(c) 35 à 95 % en poids d'un alcool en C₂ à C₄ et
(d) 0,5 à 65 % en poids d'eau.

4. Composition désinfectante suivant l'une des revendications précédentes, **caractérisée en ce qu'**elle contient :
(a) 0,6 à 2 % en poids, de préférence 1 à 2 % en poids de diacétate de chlorhexidine et/ou de digluconate de chlorhexidine,
(b) 0,01 à 3 % en poids, de préférence 0,5 à 3 % en poids, d'acide benzoïque ou d'un sel d'acide benzoïque,
(c) 25 à 75 % en poids, de préférence 30 à 60 % en poids, de l'alcool et
(d) 25 à 70 % en poids, de préférence 40 à 70 % en poids, d'eau.

5. Composition désinfectante suivant l'une des revendications précédentes, **caractérisée en ce qu'**elle contient en outre :
(g) 0 à 3 % en poids d'édulcorants.

6. Composition désinfectante suivant l'une des revendications précédentes, **caractérisée en ce qu'**elle contient en outre :
(f) 0,02 à 4 % en poids de thymol et/ou de menthol comme huiles essentielles/arômes de fruits.

7. Composition désinfectante suivant l'une des revendications précédentes, **caractérisée en ce qu'**elle contient en outre :
(e) 0,01 à 8 % en poids, de préférence 1 à 5 % en poids, d'un ou plusieurs agents tensio-actifs non ioniques.

8. Composition désinfectante suivant la revendication 7, **caractérisée en ce que** l'agent tensio-actif (e) est un alkyl-/alcényl-glucoside, un alcool alkoxylé, un acide carboxylique alkoxylé, un ester de sorbitanne, un dérivé polyéthoxylé d'un ester de sorbitanne, un ester de polyglycérol, un ester d'un acide gras avec un alcool polyhydroxylique et/ou un polyalkylèneglycol.

9. Composition désinfectante suivant l'une des revendications 1 à 7, **caractérisée en ce qu'**elle contient :
(e) 1 à 3 % en poids d'alkyl-/alcényl-glucosides comme agent tensio-actif
(f) 2 à 4 % en poids de thymol et/ou de menthol comme huiles essentielles/arômes de fruits.

10. Composition désinfectante suivant l'une des revendications 7 à 9, **caractérisée en ce que** l'agent tensio-actif (e) est un alkyl-/alcényl-glucoside ayant un poids moléculaire moyen de 250 à 1000 g/mole, de préférence de 300 à 500 g/mole et présente au moins un reste hydrocarboné ayant 6 à 20, de préférence 8 à 16 atomes de carbone.

11. Composition désinfectante suivant l'une des revendications précédentes, **caractérisée en ce que** la composition hydroalcoolique présente un pH de 5 à 8, de préférence d'environ 7.

12. Composition désinfectante suivant l'une des revendications précédentes, **caractérisée en ce qu'**elle contient comme alcool (c) de l'éthanol, du n-propanol et/ou de l'isopropanol.

13. Composition désinfectante suivant l'une des revendications précédentes, **caractérisée en ce qu'**elle contient 2,5 à 4,0 % en poids de menthol.

14. Composition, **caractérisée en ce qu'**elle contient la composition désinfectante suivant l'une des revendications précédentes en association avec 10 à 90 % en poids d'un agent propulseur ou 0,1 à 10 % en poids d'un agent épaississant.

15. Objets convenant à des fins d'hygiène dentaire, de prothèse dentaire ou d'hygiène buccale, en particulier au brossage des dents, qui doivent être introduits plus d'une fois dans la cavité buccale, imprégnés d'une composition désinfectante suivant l'une des revendications 1 à 13 ou d'une composition suivant la revendication 14.

16. Utilisation de la composition désinfectante suivant l'une des revendications 1 à 13 ou de la composition suivant la revendication 14 pour la désinfection d'objets qui, aux fins de l'hygiène dentaire, de la prothèse dentaire ou de l'hygiène buccale, en particulier du brossage des dents, sont introduits plus d'une fois dans la cavité buccale.
